# EUROPEAN PATENT APPLICATION

(11) **EP 4 781 965 A1**
(43) Date of publication of application: **29.07.2026**
(21) Application number: 26154519.8
(22) Date of filing: 27.01.2026
(51) Int. Cl.: A61G 7/10, A61L 2/10

(54) **PATIENT LIFT WITH ULTRAVIOLET RADIATION CONFIGURATION**

(30) Priority: 28.01.2025 US 202563750512 P
(71) Applicant: Amico Mobility Solutions Corporation, Richmond Hill, ON L4B 1G6 (CA)
(72) Inventor: BENSON, Wayne, Ontario, L4B 1G6 (CA); ABBASI, Habib-Ur Rehman, Ontario, L4B 1G6 (CA)
(74) Representative: Nederlandsch Octrooibureau

(57) **Abstract**

Disclosed herein is a patient lift, comprising a housing; an ultraviolet (UV) radiation configuration, mounted to the housing, and configured to emit UV radiation into a radiation zone; a spool, disposed in the housing; a strap, connected to the spool, and extending through the radiation zone such that a portion of the strap is disposed in the radiation zone; a drive unit, operably coupled to the spool such that the spool is drivable by the drive unit to displace the strap through the radiation zone; and a controller operably coupled to the UV radiation configuration to adjust a characteristic of the emitted UV radiation, and operably coupled to the drive unit to adjust the displacement of the strap through the radiation zone. While the UV radiation configuration is emitting UV radiation into the radiation zone, the portion of the strap in the radiation zone is exposed to the UV radiation.

## Description

### FIELD

The present application relates to a patient lift, and in particular, to a patient lift configured to expose a strap and a carry bar of the patient lift to ultraviolet radiation.

### BACKGROUND

A patient lift is configured to assist in the displacement of a patient who may have limited mobility. Existing patient lifts include a strap that is connectible to a carry bar, which is connectible to a patient supporter such as a sling or a seat, upon which the patient may be supported. While the patient is supported on the patient supporter, the strap is extendible or retractable to displace the patient. It is desirable to disinfect the strap and the carry bar for enhancing safety and hygiene and for promoting infection control standards, particularly in healthcare and medical environments.

### SUMMARY

In one aspect, there is provided a patient lift, comprising: a housing; an ultraviolet (UV) radiation configuration, mounted to the housing, and configured to emit UV radiation into a radiation zone; a spool, disposed in the housing; a strap, connected to the spool, and extending through the radiation zone such that a portion of the strap is disposed in the radiation zone; a drive unit, operably coupled to the spool such that the spool is drivable by the drive unit for winding the strap onto the spool, and further drivable by the drive unit for unwinding the strap from the spool, such that the strap is displaceable through the radiation zone; an energy source operably coupled to the drive unit to provide energy to the drive unit; a controller operably coupled to the UV radiation configuration to adjust a characteristic of the UV radiation emitted by the UV radiation configuration into the radiation zone, and further operably coupled to the drive unit to adjust the displacement of the strap through the radiation zone; wherein: while the UV radiation configuration is emitting UV radiation into the radiation zone, the portion of the strap that is disposed in the radiation zone is exposed to the UV radiation.

In another aspect, there is provided a patient lift, comprising: a housing; an ultraviolet (UV) radiation configuration, mounted to the housing, and configured to emit UV radiation into a radiation zone; a spool, disposed in the housing; a carry bar, configured to connect to a patient supporter; a strap, connected to the spool and further connected to the carry bar; a drive unit, operably coupled to the spool such that the spool is drivable by the drive unit for winding the strap onto the spool, and further drivable by the drive unit for unwinding the strap from the spool, such that the carry bar is displaceable, relative to the radiation zone, for effectuating disposition of the carry bar in the radiation zone; an energy source operably coupled to the drive unit to provide energy to the drive unit; a controller operably coupled to the UV radiation configuration to adjust a characteristic of the UV radiation emitted by the UV radiation configuration into the radiation zone, and further operably coupled to the drive unit to adjust the displacement of the carry bar, relative to the radiation zone; wherein: while the UV radiation configuration is emitting UV radiation into the radiation zone and the carry bar is disposed in the radiation zone, the carry bar is exposed to the UV radiation.

In another aspect, there is provided a patient lift configuration, comprising: an ultraviolet (UV) radiation configuration, mounted to a building structure, and configured to emit UV radiation into a radiation zone; a patient lift, comprising: a housing; a spool, disposed in the housing; a strap, connected to the spool, and extending through the radiation zone such that a portion of the strap is disposed in the radiation zone; a drive unit, operably coupled to the spool such that the spool is drivable by the drive unit for winding the strap onto the spool, and further drivable by the drive unit for unwinding the strap from the spool, such that the strap is displaceable through the radiation zone; an energy source operably coupled to the drive unit to provide energy to the drive unit; a controller operably coupled to the UV radiation configuration to adjust a characteristic of the UV radiation emitted by the UV radiation configuration into the radiation zone, and further operably coupled to the drive unit to adjust the displacement of the strap through the radiation zone; wherein: while the UV radiation configuration is emitting UV radiation into the radiation zone, the portion of the strap that is disposed in the radiation zone is exposed to the UV radiation.

In another aspect, there is provided a patient lift configuration, comprising: an ultraviolet (UV) radiation configuration, mounted to a building structure, and configured to emit UV radiation into a radiation zone; a patient lift, comprising: a housing; a spool, disposed in the housing; a carry bar, configured to connect to a patient supporter; a strap, connected to the spool and further connected to the carry bar; a drive unit, operably coupled to the spool such that the spool is drivable by the drive unit for winding the strap onto the spool, and further drivable by the drive unit for unwinding the strap from the spool, such that the carry bar is displaceable, relative to the radiation zone, for effectuating disposition of the carry bar in the radiation zone; an energy source operably coupled to the drive unit to provide energy to the drive unit; a controller operably coupled to the UV radiation configuration to adjust a characteristic of the UV radiation emitted by the UV radiation configuration into the radiation zone, and further operably coupled to the drive unit to adjust the displacement of the carry bar, relative to the radiation zone; wherein: while the UV radiation configuration is emitting UV radiation into the radiation zone and the carry bar is disposed in the radiation zone, the carry bar is exposed to the UV radiation.

Other aspects will be apparent from the description and drawings provided herein.

### BRIEF DESCRIPTION OF DRAWINGS

In the figures, which illustrate example embodiments,
Figure 1 is a perspective view of an example embodiment of a patient lift;
Figure 2 is a schematic diagram depicting the software structure of the patient lift of Figure 1;
Figure 3 is another perspective view of the patient lift of Figure 1;
Figure 4 is a cross-sectional view of the patient lift of Figure 3, taken along line 4-4 as depicted in Figure 3;
Figure 5 is a cross-sectional view of the patient lift of Figure 3, taken along line 4-4 as depicted in Figure 3, without the strap;
Figure 6 is a cross-sectional view of the patient lift of Figure 3, taken along line 6-6 as depicted in Figure 3;
Figure 7 is a cross-sectional view of the patient lift of Figure 3, taken along line 6-6 as depicted in Figure 3, without the strap;
Figure 8 is a front view of an example embodiment of a user interface;
Figure 9 is a schematic diagram of another example embodiment of a patient lift; and
Figure 10 is a schematic diagram of a patient lift configuration.

### DETAILED DESCRIPTION

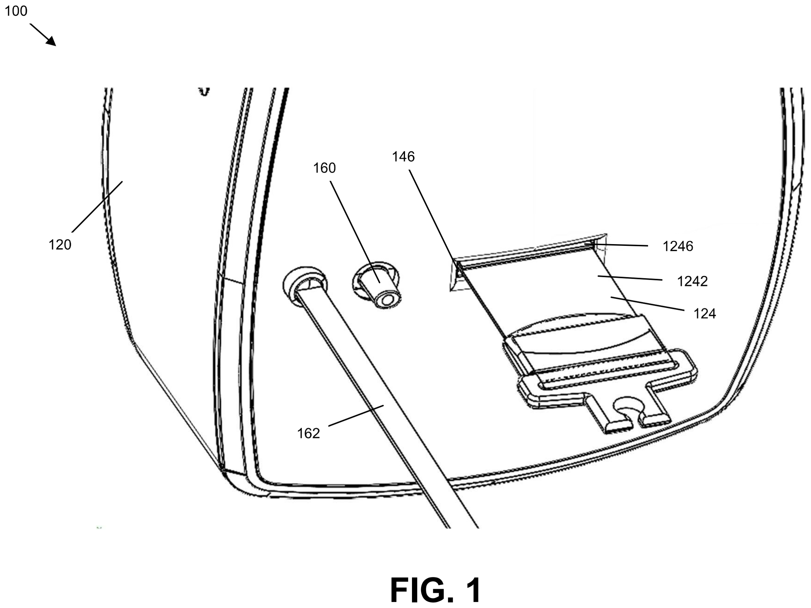

Disclosed herein in a patient lift 100 that includes an ultraviolet (UV) radiation configuration 140 that is configured to emit UV radiation into a radiation zone 142 that is defined by the UV radiation configuration 140 for disinfecting and sanitizing a component of the patient lift 100. In some embodiments, for example, as depicted in Figure 1, a strap 124 of the patient lift 100 extends through a radiation zone 142, such that a portion of the strap 124 is disposed in the radiation zone 142. The strap 124 is windable onto a spool 122 and unwindable from the spool 122. The strap 124 is displaceable through the radiation zone 142 by winding the strap 124 onto the spool 122 or unwinding the strap 124 from the spool 122. The characteristic of the UV radiation emitted by the UV radiation configuration 140 (e.g. wavelength and intensity of the emitted UV radiation) and the displacement of the strap 124 through the radiation zone 142 can be adjusted. While the UV radiation configuration 140 is emitting UV radiation into the radiation zone 142, the portion of the strap 124 that is disposed in the radiation zone 142 is exposed to the UV radiation.

In some embodiments, for example, there is a need for effective disinfection solutions, for example, to effectuate microbial reduction and reduction of pathogen viability. Such a need is particularly present in healthcare settings, where the risk of infection transmission is high. UV disinfection has emerged as a method for eliminating pathogens, including bacteria, viruses, and fungi, from various surfaces. In some embodiments, for example, by exposing pathogens and microorganisms to UV radiation, the DNA and RNA of the pathogens and microorganisms are disrupted. In some embodiments, for example, such disruption of the DNA and RNA of the pathogens and microorganisms renders the pathogens and microorganisms inactive and unable to reproduce, which, in some embodiments, for example, is effective for inactivating a broad spectrum of microorganisms and pathogens. In some embodiments, for example, the inactivation of microorganisms and pathogens is with effect that the surface, on which the pathogens and microorganisms are disposed, is disinfected and sanitized, at least with respect to the pathogens and microorganisms.

In some embodiments, for example, by exposing the portion of the strap 124 that is disposed in the radiation zone 142 to the UV radiation emitted by the UV radiation configuration 140, the DNA and RNA of the pathogens and microorganisms disposed on the portion of the strap 124 are disrupted, with effect that the pathogens and microorganisms become inactive and are unable to reproduce, which, in some embodiments, for example, effectuates disinfecting and sanitizing of the portion of the strap 124 that is exposed to the UV radiation. The strap 124 can be displaced through the radiation zone 142 to expose other portions of the strap 124 to UV radiation.

By adjusting the UV radiation wavelength and intensity of the UV radiation emitted by the UV radiation configuration 140 to the portion of the strap 124 and the duration of exposure of UV radiation to the portion of the strap 124, the dosage of UV radiation emitted to the portion of the strap 124 can be controlled, and therefore, the disinfection and sanitation of the strap 124 can be controlled. In some embodiments, for example, the patient lift 100 is configured to deliver the optimal dosage of UV radiation required to achieve effective disinfection of the strap 124 without damaging the patient lift 100 itself or posing risks to users.

In some embodiments, for example, by disinfecting and sanitizing the strap 124, a safer and more hygienic environment is provided to users, such as patients and healthcare providers.

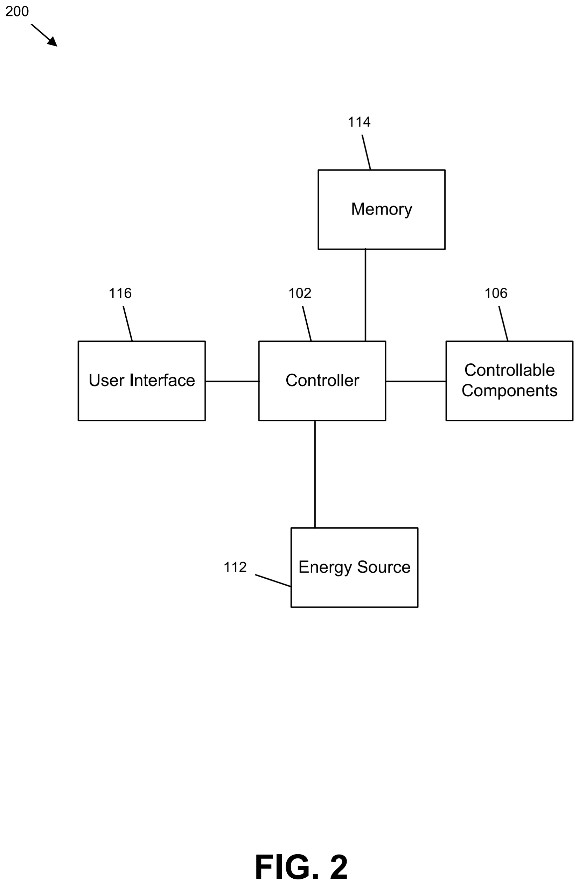

Figure 2 is a schematic diagram showing the software structure 200 of the patient lift 100 in some embodiments. In some embodiments, for example, the patient lift 100 comprises a control module or a controller 102 disposed in operable communication with controllable components 106, a user interface 116, an energy source 112, and a memory 114, as described in greater detail below.

In some embodiments, for example the patient lift 100 comprises a plurality of components including, e.g., a controller 102, memory 114 (volatile and/or non-volatile memory, e.g., RAM, ROM, EEPROM, and/or the like), non-removable or removable memory (e.g., hard disk drives, CD-ROMs, DVDs, solid-state memory, flash memory, and/or the like), networking components for connecting to a network, a plurality of controllable components 106 including a UV radiation configuration 140 and a drive unit 126, and a system bus coupling the various components to the controller 102.

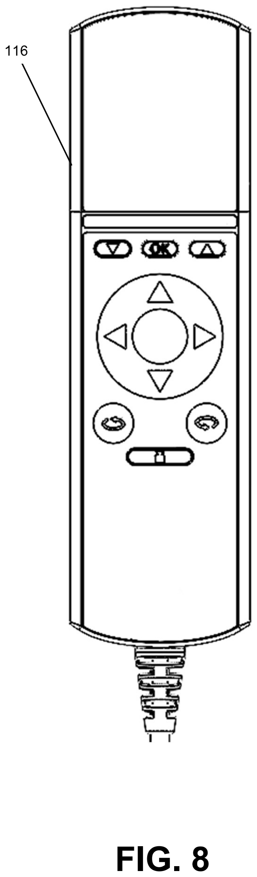

In some embodiments, for example the patient lift 100 comprises a user interface 116 to effectuate interaction between the patient lift 100 and a user. In some embodiments, for example, the user interface 116 includes a display such as a display screen or a touchscreen, a control panel, one or more input devices such as buttons or a hand control unit, a keyboard and a computer mouse, other input/output devices such as a microphone, a speaker, a printer, a scanner, and/or the like. Figure 8 depicts an example embodiment of the user interface 116, wherein the user interface 116 is a hand control unit. As depicted in Figure 8, the hand control unit comprises buttons for an operator to provide inputs for controlling the function of the patient lift 100. In some embodiments, for example, the hand control unit as depicted in Figure 8 is operably coupled to the housing 120 via a cover strain relief 160 and a conduit.

In some embodiments, the controller 102 includes a processor or a central processing unit (CPU) that is operably coupled to a memory 114 such as a ROM, RAM, persistent memory, or flash memory for storing data, and input or output peripherals. In some embodiments, for example, the controller 102 functions as a central controller for controlling all of the communications of the patient lift 100. In some embodiments, for example, the controller 102 further functions as a central controller for controlling the communications between the patient lift 100 and an external server or user equipment, such as a computer, laptop, smart device, a control panel in a control room, and the like.

In some embodiments, for example, the processor is one or more single-core or multiple-core computing processors such as INTEL^{®} microprocessors (INTEL is a registered trademark of Intel Corp., Santa Clara, CA, USA), AMD^{®} microprocessors (AMD is a registered trademark of Advanced Micro Devices Inc., Sunnyvale, CA, USA), ARM^{®} microprocessors (ARM is a registered trademark of Arm Ltd., Cambridge, UK) manufactured by a variety of manufactures such as Qualcomm of San Diego, California, USA, under the ARM^{®} architecture, or the like.

In some embodiments, for example, the processor is one or more real-time processors, programmable logic controllers (PLCs), microcontroller units (MCUs), µ-controllers (UCs), specialized or customized processors or controllers using e.g., field-programmable gate array (FPGA) or application-specific integrated circuit (ASIC) technologies, and/or the like.

The controller 102 communicates with the controllable components 106, the user interface 116, the energy source 112, and the memory 114. In some embodiments, the controller 102 receives data or instructions, saves them to a memory, and processes the received data or instructions. The data or instructions may be real time or historical data or instructions. In some embodiments, the controller 102 processes the data or instructions by, for example, comparing them with one or more preset thresholds.

In some embodiments, the controller 102 is configured to control the functioning or operation of the patient lift 100. In some embodiments, for example, based on the data from a component of the patient lift 100, such as a controllable component 106 or the energy source 112, the controller 102 sends a control command to the user interface 116 to render a graphic representative of the data, or sends a control command to one or more of the controllable components 106 to operate the patient lift 100.

In some embodiments, the patient lift 100 includes a user interface 116 operably coupled to the controller 102 to receive instructions from a user, and to communicate information to a user. In some embodiments, for example, the user interface 116 includes an input device, such as user equipment, a keyboard, mouse, camera, touch screen, one or more buttons, a hand control unit, a microphone, a control panel, and the like. In some embodiments, for example, the user interface 116 incudes an output device, such as a display screen or touch screen, a speaker, a printer, a scanner, the hand control unit, and the like. In some embodiments, for example, the user interface 116 is configured to operably couple the controller 102 to an external input device, such as a user device (e.g. computer, smartphone, etc.), for the controller 102 to receive instructions from the external input device. In some embodiments, for example, the user interface 116 is configured to operably couple the controller 102 to an external output device to communicate information to the external output device. In some embodiments, the controller 102 is configured to send a control command to the user interface 116 for displaying a graphical representation of data that is received by the controller 102. In some embodiments, the user interface 116, via input from a user, is configured to send a control command to the controller 102 for controlling the patient lift 100. In some embodiments, for example, a user can input a control command to the controller 102 via the user interface 116 to wind the strap 124 to the spool 122 or unwind the strap 124 from the spool 122, adjust the characteristics of the UV radiation emitted from the UV radiation configuration 140, adjust the displacement of the strap 124 through the radiation zone 142, select the environment in which the patient lift 100 is disposed, select the operating mode of the patient lift 100, and the like.

In some embodiments, for example, the controller 102 sends a control command to the user interface 116 to generate a graphical representation of the operating status of the patient lift 100. In some embodiments, for example, the controller 102 sends a control command to the user interface 116 to generate a graphical representation of a menu of options, and the user can input a control command using the user interface 116 to select an option from the menu, and the user interface 116 sends the control command to the controller 102 to control the operation of the patient lift 100. In some embodiments, for example, the menu of options includes a list of possible environments in which the patient lift 100 is disposed. In some embodiments, for example, the menu of options includes a list of operating modes of the patient lift 100.

The controller 102 receives instructions from a user via the user interface 116 or data from one or more components of the patient lift 100 to make a plurality of decisions, such as the speed at which the strap 124 is to be wound onto the spool 122 or unwound from the spool 122, the characteristic of the UV radiation to be emitted by the UV radiation configuration 140, whether the same or different patients are being lifted by the patient lift 100, the environment of the patient lift 100, the operating mode of the patient lift 100, and the like, based on the received user instructions or data. The controller 102 then instructs the controllable components 106 to function accordingly.

In some embodiments, for example, the patient lift 100 includes an energy source 112 for providing energy to the components of the patient lift 100, such as the controller 102, memory 114, user interface 116, and controllable components 106. In some embodiments, for example, the energy source is an electrical energy source, such as a battery. In some embodiments, for example, the energy source is a fuel cell. In some embodiments, the patient lift 100 is operably coupled with an external energy source, such as a portable battery, portable generator, external battery, and the like. In some embodiments, for example, the patient lift 100 is wirelessly connectable to the external energy source for wirelessly energizing the patient lift 100.

In some embodiments, for example, the controller 102 is operably coupled to the energy source 112 for adjusting the amount of energy provided to the controllable components 106.

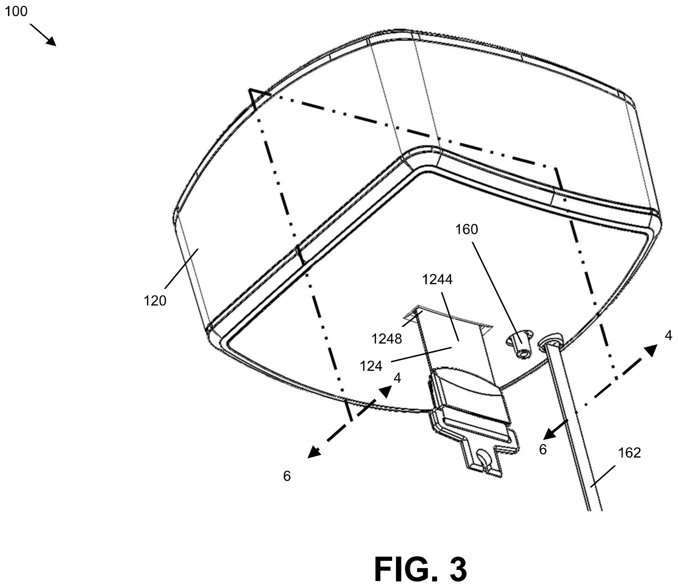

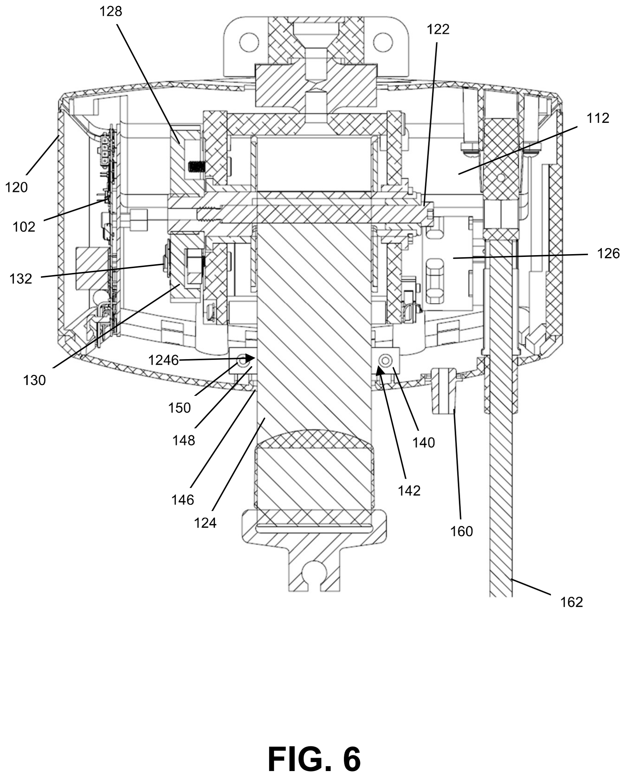

Figure 1, and Figure 3 to Figure 7 depict an example embodiment of the patient lift 100.

In some embodiments, for example, the patient lift 100 includes a housing 120. In some embodiments, for example, the patient lift 100 further includes the UV radiation configuration 140, which is mounted to the housing 120, and is configured to emit UV radiation into a radiation zone 142. In some embodiments, for example, the radiation zone 142 is defined by the UV radiation configuration 140. In some embodiments, for example, the mounting of the UV radiation configuration 140 to the housing 120 is such that the UV radiation configuration 140 is disposed in the housing 120. In some embodiments, for example, at least a portion of the radiation zone 142 is defined in the housing 120. In some embodiments, for example, the entirety of the radiation zone 142 is defined in the housing 120. In some embodiments, for example, by disposing the UV radiation configuration 140 in the housing 120, the UV radiation emitted from the UV radiation configuration 140 is opposed (e.g. prevented) by the housing 120 from being emitted outside of the housing 120, thereby improving patient and operator safety. In some embodiments, for example, the UV radiation that is emitted by the UV radiation configuration 140 is emitted into the radiation zone 142. In some embodiments, for example, the radiation zone 142 is a zone in which UV radiation that is emitted by the UV radiation configuration 140 is received.

In some embodiments, for example, the patient lift 100 further includes a spool 122, which is disposed in the housing 120. In some embodiments, for example, the spool 122 is connected to the housing 120 such that the spool 122 is rotatable, relative to the housing 120.

In some embodiments, for example, the patient lift 100 further includes a strap 124. The strap 124 is connected to the spool 122. In some embodiments, for example, the strap 124 extends through the radiation zone 142, such that a portion of the strap 124 is disposed in the radiation zone 142. In some embodiments, for example, the strap 124 is configured to connect to a patient supporter such as a sling or a seat. In some embodiments, for example, the strap 124 is configured to connect to a carry bar 210, which is configured to connect to the patient supporter.

In some embodiments, for example, the patient lift 100 further includes the drive unit 126. In some embodiments, for example, the drive unit 126 is operably coupled to the spool 122 such that the spool 122 is drivable by the drive unit 126 for winding the strap 124 onto the spool 122, with effect that the strap 124 is retracted, and further drivable by the drive unit 126 for unwinding the strap 124 from the spool 122, with effect that the strap 124 is extended. In some embodiments, for example, the drivability of the spool 122 by the drive unit 126 is such that the strap 124 is displaceable through the radiation zone 142. In some embodiments, for example, the drive unit 126 is a motor. In some embodiments, for example, the drive unit 126 is an electric motor. In some embodiments, for example, the drive unit 126 is a planetary motor. In some embodiments, for example, while the strap 124 is connected to a patient supporter, and the patient is supported on the patient supporter, the strap 124 is extendible, by unwinding the strap 124 from the spool 122, or retractable, by winding the strap 124 onto the spool 122, to displace the patient (e.g. to lift or lower the patient).

In some embodiments, for example, the patient lift 100 further includes a transmission configuration 128 to effectuate the operable coupling of the drive unit 126 and the spool 122, such that the winding of the strap 124 onto the spool 122 and the unwinding of the strap 124 from the spool 122 by the drive unit 126 is effectuatable by the transmission configuration 128. In this respect, the transmission configuration 128 is operably coupled to the drive unit 126, and further operably coupled to the spool 122. In some embodiments, for example, the transmission configuration 128 includes one or more transmission components 130, such as a gear, a wheel, and the like. In some embodiments, for example, one or more transmission components 130 is operably coupled to the spool 122 such that rotation of the one or more transmission components 130 effectuates rotation of the spool 122. In some embodiments, for example, the transmission configuration 128 includes a drive shaft 132 that is drivable by the drive unit 126, for effectuating rotation of the one or more transmission components 130. In some embodiments, for example, the transmission configuration 128 includes a chain, a belt, and the like, for operably coupling the one or more transmission components 130.

In some embodiments, for example, the patient lift 100 further includes the energy source 112. In some embodiments, for example, the energy source 112 is operably coupled to the drive unit 126 to provide energy to the drive unit 126. In some embodiments, for example, the energy source 112 is operably coupled to the UV radiation configuration 140 to provide energy to the UV radiation configuration 140.

In some embodiments, for example, the patient lift 100 further includes the controller 102. The controller 102 is operably coupled to the UV radiation configuration 140 to adjust a characteristic of the UV radiation emitted by the UV radiation configuration 140 into the radiation zone 142. The controller 102 is further operably coupled to the drive unit 126 to adjust the displacement of the strap 124 through the radiation zone 142.

In some embodiments, for example, while the UV radiation configuration 140 is emitting UV radiation into the radiation zone 142, the portion of the strap 124 that is disposed in the radiation zone 142 is exposed to the UV radiation emitted by the UV radiation configuration 140.

In some embodiments, for example, the controller 102 is configured to determine a usage frequency of the patient lift 100 based at least in part on the energy provided by the energy source 112 to the drive unit 126.

In some embodiments, for example, the adjusting of the characteristic of the UV radiation is based at least in part on the usage frequency of the patient lift 100. In some embodiments, for example, based on a determination that the usage frequency is above a predetermined threshold, the intensity of the UV radiation emitted from the UV radiation configuration 140 is increased. In some embodiments, for example, based on a determination that the usage frequency is below a predetermined threshold, the intensity of the UV radiation emitted from the UV radiation configuration 140 is decreased.

In some embodiments, for example, the adjusting of the displacement of the strap 124 through the radiation zone 142 is based at least in part on the usage frequency of the patient lift 100. In some embodiments, for example, based on a determination that the usage frequency is above a predetermined threshold, the displacement of the strap 124 through the radiation zone 142 is decreased. In some embodiments, for example, based on a determination that the usage frequency is below a predetermined threshold, the displacement of the strap 124 through the radiation zone 142 is increased.

In some embodiments, for example, the controller 102 is configured to determine that different patients are using the patient lift 100 based at least in part on the energy provided by the energy source 112 to the drive unit 126. In some embodiments, for example, the determination that different patients are using the patient lift 100 is based on a correlation of the amount of energy provided by the energy source 112 to the drive unit 126 and the weight of a patient, which, in some embodiments, for example, differs between patients.

In some embodiments, for example, the adjusting of the characteristic of the UV radiation emitted by the UV radiation configuration 140 is based at least in part on the determination that different patients are using the patient lift 100. In some embodiments, for example, based on a determination that the number of patients using the patient lift 100 is above a predetermined threshold, the intensity of the UV radiation emitted from the UV radiation configuration 140 is increased. In some embodiments, for example, based on a determination that the number of patients using the patient lift 100 is below a predetermined threshold, the intensity of the UV radiation emitted from the UV radiation configuration 140 is decreased. In some embodiments, for example, based on a determination that different patients are using the patient lift 100, the intensity of the UV radiation emitted from the UV radiation configuration 140 is increased. In some embodiments, for example, based on a determination that the same patient is using the patient lift 100, the intensity of the UV radiation emitted from the UV radiation configuration 140 is decreased.

In some embodiments, for example, the adjusting of the displacement of the strap 124 through the radiation zone 142 is based at least in part on the determination that different patients are using the patient lift 100. In some embodiments, for example, based on a determination that the number of patients using the patient lift 100 is above a predetermined threshold, the displacement of the strap 124 through the radiation zone 142 is decreased. In some embodiments, for example, based on a determination that the number of patients using the patient lift 100 is below a predetermined threshold, the displacement of the strap 124 through the radiation zone 142 is increased. In some embodiments, for example, based on a determination that the different patients are using the patient lift 100, the displacement of the strap 124 through the radiation zone 142 is decreased. In some embodiments, for example, based on a determination that the patient lift 100 being used by the same patient, the displacement of the strap 124 through the radiation zone 142 is increased.

In some embodiments, for example, the patient lift 100 further includes the user interface 116. The user interface 116 is configured to receive an input representative of an environment of the patient lift 100, for example, from a user via a hand control unit, and communicating the input to the controller 102.

In some embodiments, for example, the adjusting of the characteristic of the UV radiation emitted by the UV radiation configuration 140 is based at least in part on the environment of the patient lift 100. In some embodiments, for example, based on an input representative of an environment that has relatively high sanitation requirements (e.g. operating room), the intensity of the UV radiation emitted from the UV radiation configuration 140 is increased. In some embodiments, for example, based on an input representative of an environment that has relatively low sanitation requirements (e.g. patient room), the intensity of the UV radiation emitted from the UV radiation configuration 140 is decreased.

In some embodiments, for example, the adjusting of the displacement of the strap 124 through the radiation zone 142 is based at least in part on the environment of the patient lift 100. In some embodiments, for example, based on an input representative of an environment that has relatively high sanitation requirements (e.g. operating room), the displacement of the strap 124 through the radiation zone 142 is decreased. In some embodiments, for example, based on an input representative of an environment that has relatively low sanitation requirements (e.g. patient room), the displacement of the strap 124 through the radiation zone 142 is increased.

In some embodiments, for example, the user interface 116 is configured to receive an input representative of an operating mode of the patient lift 100, for example, via a hand control unit, and communicating the input to the controller 102. In some embodiments, for example, the adjusting of the characteristic of the UV radiation emitted by the UV radiation configuration 140 is based at least in part on the operating mode of the patient lift 100. In some embodiments, for example, the adjusting of the displacement of the strap 124 through the radiation zone 142 is based at least in part on the operating mode of the patient lift 100.

In some embodiments, for example, an operating mode of the patient lift 100 includes a continuous mode or usage mode. In some embodiments, for example, the continuous mode can be selected via the user interface 116. While the patient lift 100 is operating in the continuous mode, the UV radiation configuration 140 emits UV radiation continuously, for example, while the patient lift 100 is in operation or is able to be operated, such that the portion of the strap 124 that is disposed in the radiation zone 142 is exposed to the UV radiation continuously. In some embodiments, for example, while the patient lift 100 is operating in the continuous mode, the continuous emission of UV radiation on the portion of the strap 124 in the radiation zone 142 effectuates ongoing disinfection of the portion of the strap 124 in the radiation zone 142. In some embodiments, for example, the operation of the patient lift 100 in the continuous mode is particularly useful in high-traffic environments where the patient lift 100 is frequently operated. In some embodiments, for example, while the patient lift 100 is in operation or is able to be operated, the patient lift 100 is disposed in the continuous mode. In some embodiments, for example, while the patient lift 100 is in operation or is able to be operated, the speed of winding of the strap 124 onto the spool 122 and the unwinding of the strap 124 from the spool 122 is expected to be faster, such that, in some embodiments, for example, the displacement of the strap 124 through the radiation zone 142 is increased, and the winding of the strap 124 onto the spool 122 and the unwinding of the strap 124 from the spool 122 is expected to occur more frequently. Accordingly, in some embodiments, for example, there is reduced exposure of the portion of the strap 124 in the radiation zone 142 to the UV radiation emitted by the UV radiation configuration 140. As such, in some embodiments, for example, while the patient lift 100 is disposed in the continuous mode, the intensity of the UV radiation emitted by the UV radiation configuration 140 is increased, to mitigate the reduced exposure of the portion of the strap 124 in the radiation zone 142 to the UV radiation emitted by the UV radiation configuration 140.

In some embodiments, for example, an operating mode of the patient lift 100 includes a disinfection mode. In some embodiments, for example, the disinfection mode can be selected via the user interface 116. In some embodiments, for example, the disinfection mode can be activated during periods when the patient lift 100 is not in use or not operational. While the patient lift 100 is operating in the disinfection mode, the UV radiation emitted by the UV radiation configuration 140 is adjusted to have increased intensity, and the strap 124 is slowly unwound from the spool 122 and wound upon the spool 122, such that the strap 124 is slowly or intermittently displaced through the radiation zone 142, which allows increased exposure of the portion of the strap 124 that is disposed in the radiation zone 142 to the UV radiation emitted from the UV radiation configuration 140.

In some embodiments, for example, while the patient lift 100 is not in use or not operational, the speed of winding of the strap 124 onto the spool 122 and the unwinding of the strap 124 from the spool 122 is expected to be reduced or absent, and the winding of the strap 124 onto the spool 122 and the unwinding of the strap 124 from the spool 122 is expected to occur less frequently or be absent. Accordingly, in some embodiments, for example, there is increased exposure of the portion of the strap 124 in the radiation zone 142 to the UV radiation emitted by the UV radiation configuration 140. As such, in some embodiments, for example, while the patient lift 100 is disposed in the disinfection mode, the intensity of the UV radiation emitted by the UV radiation configuration 140 is increased, to expose the portion of the strap 124 in the radiation zone 142 to UV radiation of increased intensity, which, in some embodiments, for example, intensifies the disinfection of the portion of the strap 124 that is disposed in the radiation zone 142. In some embodiments, for example, while the patient lift 100 is disposed in the disinfection mode, the strap 124 is displaced through the radiation zone 142, for example, slowly or intermittently, to increase the dosage of UV radiation exposed to the strap 124, and to expose other portions of the strap 124 to UV radiation emitted by the UV radiation configuration 140.

In some embodiments, for example, the adjustable characteristic of the UV radiation emitted from the UV radiation configuration 140 includes a wavelength of the UV radiation. In some embodiments, for example, the adjusting of the wavelength of the UV radiation includes increasing the wavelength of the UV radiation. In some embodiments, for example, the adjusting of the wavelength of the UV radiation includes decreasing the wavelength of the UV radiation.

In some embodiments, for example, the UV radiation configuration 140 emits broad spectrum UV radiation. In some embodiments, for example, the UV radiation configuration 140 emits UV radiation of various wavelengths to have disinfection efficacy across a wide range of pathogens and microorganisms. In some embodiments, for example, the UV radiation configuration 140 emits Ultraviolet-C (UVC) radiation.

In some embodiments, for example, the adjustable characteristic of the UV radiation emitted from the UV radiation configuration 140 includes an intensity of the UV radiation. In some embodiments, for example, the adjusting of the intensity of the UV radiation includes increasing the intensity of the UV radiation. In some embodiments, for example, the adjusting of the intensity of the UV radiation includes decreasing the intensity of the UV radiation.

In some embodiments, for example, the adjusting of the displacement of the strap 124 through the radiation zone 142 is to adjust an amount of time that the portion of the strap 124 is disposed in the radiation zone 142. In some embodiments, for example, the adjusting of the displacement of the strap 124 through the radiation zone 142 is to adjust the exposure of UV radiation emitted by the UV radiation configuration 140 to the portion of the strap 124 disposed in the radiation zone 142. In some embodiments, for example, the adjusting of the displacement of the strap 124 through the radiation zone 142 is such that the displacement of the strap 124 through the radiation zone 142 is increased. In some embodiments, for example, the adjusting of the displacement of the strap 124 through the radiation zone 142 is such that the displacement of the strap 124 through the radiation zone 142 is decreased. In some embodiments, for example, the adjusting of the displacement of the strap 124 through the radiation zone 142 is such that there is an absence of displacement of the strap 124 through the radiation zone 142. In some embodiments, for example, the adjusting of the displacement of the strap 124 through the radiation zone 142 is such that another portion of the strap 124 is disposed in the radiation zone 142.

In some embodiments, for example, the UV radiation configuration 140 comprises a UV radiation source 144 for emitting UV radiation.

In some embodiments, for example, adjusting the wavelength of the UV radiation emitted from the UV radiation configuration 140 includes adjusting the wavelength emitted from the UV radiation source 144. In some embodiments, for example, adjusting the intensity of the UV radiation emitted from the UV radiation configuration 140 includes adjusting the intensity emitted from the UV radiation source 144.

In some embodiments, for example, the UV radiation source 144 is a first UV radiation source 144, and the UV radiation configuration 140 further comprises a second UV radiation source 144 for emitting UV radiation.

In some embodiments, for example, adjusting the wavelength of the UV radiation emitted from the UV radiation configuration 140 includes adjusting the wavelength of the UV radiation emitted from one or more UV radiation sources 144. In some embodiments, for example, the wavelength of UV radiation emitted from a first UV radiation source 144 is the same as the wavelength of UV radiation emitted from a second UV radiation source 144. In some embodiments, for example, the wavelength of UV radiation emitted from the first UV radiation source 144 and wavelength of UV radiation emitted from the second UV radiation source 144 are different. In some embodiments, for example, adjusting the wavelength of the UV radiation emitted from the UV radiation configuration 140 includes adjusting the wavelength by turning on or off one or more UV radiation sources 144.

In some embodiments, for example, adjusting the intensity of the UV radiation emitted from the UV radiation configuration 140 includes adjusting the intensity of the UV radiation emitted from one or more UV radiation sources 144. In some embodiments, for example, the intensity of UV radiation emitted from the first UV radiation source 144 is the same as the intensity of UV radiation emitted from the second UV radiation source 144. In some embodiments, for example, the intensity of UV radiation emitted from the first UV radiation source 144 and intensity of UV radiation emitted from the second UV radiation source 144 are different. In some embodiments, for example, adjusting the intensity of the UV radiation emitted from the UV radiation configuration 140 includes adjusting the intensity by turning on or off one or more UV radiation sources 144.

In some embodiments, for example, the first UV radiation source 144 and the second UV radiation source 144 are disposed in a side-by-side relationship. In some embodiments, for example, the first UV radiation source 144 and the second UV radiation source 144 are disposed in opposing relationship.

In some embodiments, for example, the minimum spacing distance between the portion of the strap 124 disposed in the radiation zone 142 and the first UV radiation source 144 and the minimum spacing distance between the portion of the strap 124 disposed in the radiation zone 142 and the second UV radiation source 144 are the same. In some embodiments, for example, the minimum spacing distance between the portion of the strap 124 disposed in the radiation zone 142 and the first UV radiation source 144 and the minimum spacing distance between the portion of the strap 124 disposed in the radiation zone 142 and the second UV radiation source 144 are different.

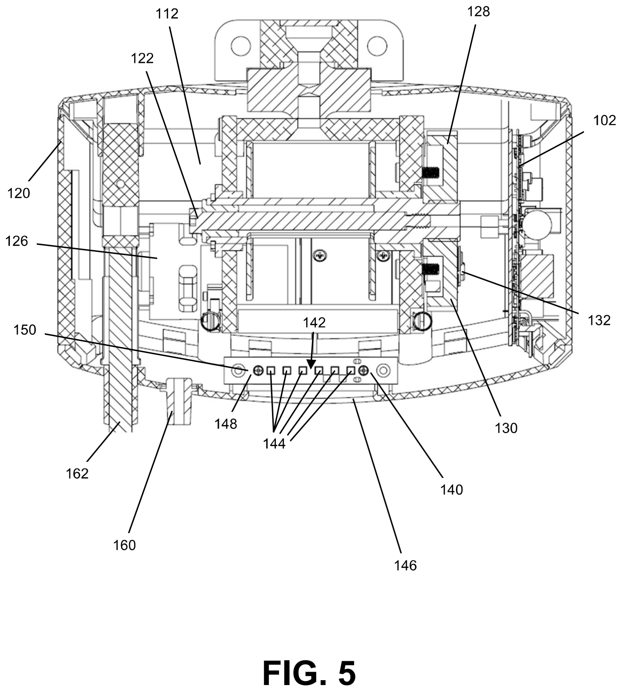

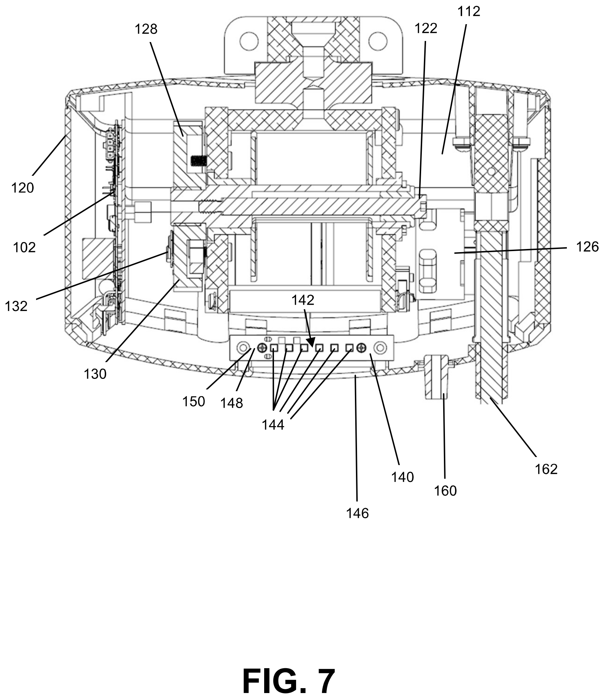

In some embodiments, for example, the patient lift 100 includes a plurality of UV radiation sources 144. As depicted in Figure 1, and Figure 3 to Figure 7, and particularly in Figure 5 and Figure 7, in some embodiments, for example, the patient lift 100 includes 12 UV radiation sources 144, with six UV radiation sources 144 disposed in a side-by-side relationship and disposed in opposing relationship, relative to another six UV radiation sources 144 disposed in a side-by-side relationship. In some embodiments, for example, the wavelength and intensity of the UV radiation emitted from the UV radiation configuration 140 are adjusted by turning on the desired UV radiation sources 144, such that the UV radiation emitted from the UV radiation sources 144 combine to generate the UV radiation of desired wavelength and intensity.

In some embodiments, for example, for each one of the UV radiation sources 144, independently, the UV radiation source 144 is a UV light source. In some embodiments, for example, for each one of the UV radiation sources 144, independently, the UV radiation source 144 is a light emitting diode (LED). In some embodiments, for example, the LED is a visible LED. In some embodiments, for example, the LED is a non-visible LED. In some embodiments, for example, for each one of the UV radiation sources 144, independently, the UV radiation source 144 is a UV bulb. In some embodiments, for example, for each one of the UV radiation sources 144, independently, the UV radiation source 144 is a UV radiation emitter.

In some embodiments, for example, the UV radiation configuration 140 is defined by a strip of UV radiation emitters (e.g. UV radiation sources 144).

In some embodiments, for example, as depicted in Figure 1, Figure 3, and Figure 4 to Figure 7, the strap 124 has a first side surface 1242 (e.g. a first broad side surface) and a second side surface 1244 (e.g. a second broad side surface) that is disposed opposite the first side surface 1242, such that the portion of the strap 124 that is disposed in the radiation zone 142 has a first side surface portion 1246 (e.g. a first broad side surface portion) and a second side surface portion 1248 (e.g. a second broad side surface portion) that is disposed opposite the first side surface portion 1246. In some embodiments, for example, a first UV radiation source 144 and the first side surface portion 1246 are disposed in opposing relationship, and a second UV radiation source 144 and the second side surface portion 1248 are disposed in opposing relationship.

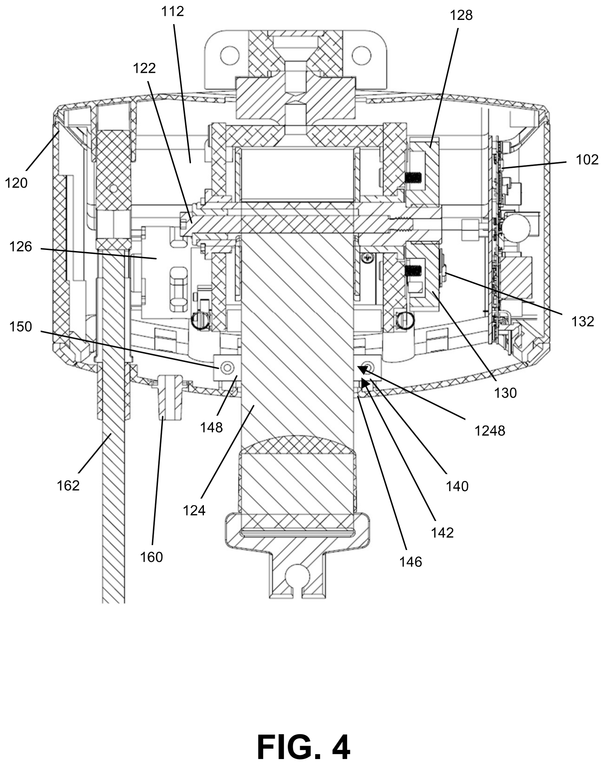

In some embodiments, for example, as depicted in Figure 4 to Figure 7, the strap 124 extends through an opening 146 defined by the housing 120. In some embodiments, for example, the opening 146 and the radiation zone 142 are disposed in alignment. In some embodiments, for example, the opening 146 and the radiation zone 142 are aligned along an alignment axis that is a linear axis. In some embodiments, for example, the housing 120, particularly the portion of the housing 120 that defines the opening 146, is frequently touched, and thus, susceptible to contamination. In some embodiments, for example, the UV radiation configuration 140 is disposed proximate to the opening 146 for exposing the portion of the housing 120 that defines the opening 146 to UV radiation. In some embodiments, for example, the portion of the housing 120 that defines the opening 146 is disposed in the radiation zone 142.

In some embodiments, for example, as depicted in Figure 4 to Figure 7, the strap 124 extends through an opening 148 defined by an opening-defining surface 150 of the UV radiation configuration 140. In some embodiments, for example, at least a portion of the opening-defining surface 150 of the UV radiation configuration 140 is disposed in the radiation zone 142, such that, while the UV radiation configuration 140 is emitting the UV radiation into the radiation zone 142, the at least a portion of the opening-defining surface 150 of the UV radiation configuration 140 is exposed to the UV radiation. In some embodiments, for example, the opening 146 and the opening 148 are disposed in alignment. In some embodiments, for example, the opening 146 and the opening 148 are aligned along an alignment axis that is a linear axis.

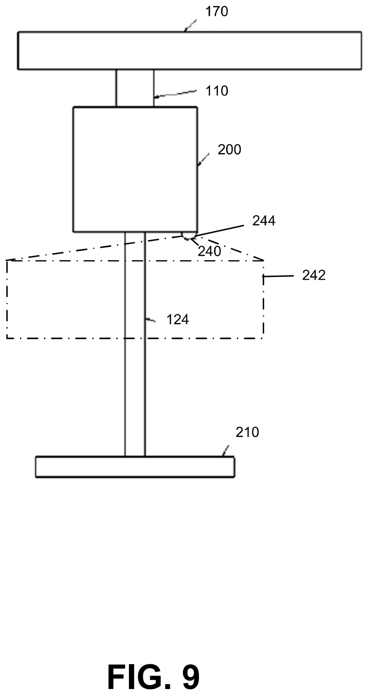

In some embodiments, for example, the patient lift 100 is configured to be connected, for example, mounted, to a ceiling, for example, of a room. In some embodiments, for example, the patient lift 100 is configured to be connected, for example, mounted, to a patient lift supporter, for example, a frame, that is configured to be supported by a reaction surface, such as a floor, for example, of a room. In some embodiments, for example, the patient lift 100 is configured to be operably coupled to a track configuration for displacing the patient lift along a first displacement axis. In some embodiments, for example, the track configuration is configured to be connected, for example, mounted, to a ceiling. As depicted in Figure 9, in some embodiments, for example, the track configuration includes a first track 110. In some embodiments, the patient lift 100 and the first track 110 are co-operatively configured such that the patient lift 100 is displaceable, relative to the first track 110, along an axis that is parallel to the longitudinal axis of the first track 110, for displacing the patient lift along the first displacement axis. In some embodiments, for example, the track configuration further includes a second track 170. In some embodiments, the first track 110 and the second track 170 are co-operatively configured such that the first track 110 is displaceable, relative to the second track 170, along an axis that is parallel to the longitudinal axis of the second track 170, for displacing the patient lift 100 along a second displacement axis, wherein the first displacement axis and the second displacement axis are disposed in a non-parallel relationship, for example, a perpendicular relationship. In some embodiments, for example, the track configuration is mountable to a room, for example, the ceiling, via mechanical fasteners (e.g. nuts and bolts, screws, and the like). In some embodiments, for example, the displaceability of the patient lift 100, relative to the track configuration, is effectuatable manually, for example, by applying a displacement force to the patient lift 100.

In some embodiments, for example, the patient lift 100 includes safety features to oppose, for example, prevent, emission of the UV radiation outside of the radiation zone 142, such that, in some embodiments, for example, the UV radiation is limited to being emitted in the radiation zone 142, for reducing the risk of accidental exposure of the UV radiation to users. In some embodiments, for example, the patient lift 100 includes an emergency pull cord 162. In some embodiments, for example, in response to pulling on the emergency pull cord 162, the controller 102 sends a control command to the UV radiation configuration 140 to stop emission of UV radiation. In some embodiments, for example, in response to pulling on the emergency pull cord 162, the controller 102 sends a control command to the drive unit 126 to stop driving of the spool 122.

In some embodiments, for example, a user can control the patient lift 100 via a hand control unit of the user interface 116, which is disposed in operable configuration with the controller 102. The user can select the mode that the patient lift 100 is to operate in, and initiate the operation of the patient lift 100. In response to the selection of the operating mode, the wavelength and intensity of the UV radiation emitted by the UV radiation configuration 140 is adjusted, and the displacement of the strap 124 through the radiation zone 142 is adjusted, for exposing the strap 124 to UV radiation emitted by the UV radiation configuration 140 having the desired characteristics for the desired duration. The user can further pause or stop the operation of the patient lift 100 via the hand control unit. In some embodiments, for example, after the operation of the patient lift 100 is initiated, a pre-determined amount of time is allowed to pass, before the UV radiation configuration 140 begins to emit UV radiation, which allows time for an operator to leave the room in which the patient lift 100 is disposed. In some embodiments, for example, the status of the patient lift 100 is displayed on the user interface 116, for example, on the hand control unit. In some embodiments, for example, the status of the operation of the patient lift 100 is displayed on the user interface 116, for example, on the hand control unit. In some embodiments, for example, an alert, for example, an audible or visible alert, is provided by the user interface 116, to indicate to a user that the UV radiation configuration 140 is, or will be, emitting UV radiation. In some embodiments, for example, while the UV radiation configuration 140 is emitting UV radiation, the controller 102 will send a control command to the UV radiation configuration 140 to stop emission of the UV radiation after a pre-determined amount of time has passed. In such embodiments, for example, the UV radiation is emitted for the predetermined amount of time to achieve the desired exposure of the strap 124 to the UV radiation, and the UV radiation is not emitted more than desired or necessary.

In some embodiments, for example, the patient lift 100 includes visible warnings, such as labels and signs, to indicate to a user or bystander that the patient lift 100 is able to emit UV radiation, and to identify potential hazards of UV radiation and appropriate precautions that can be taken. In some embodiments, for example, the visible warnings are disposed on the housing 120 of the patient lift 100.

In some embodiments, for example, the UV radiation configuration 140 is mountable on other structural components of the patient lift 100 or on an accessory that is connectible to a structural component of the patient lift 100, such as a carry bar, for providing flexibility in positioning of the UV radiation configuration 140 and radiation zone 142, relative to the strap 124, to achieve desirable exposure of the strap 124 to the emitted UV radiation, which, in some embodiments, for example, achieves desirable disinfection and sanitization of the strap 124.

Figure 9 depicts a patient lift 200 that is an alternate embodiment of the patient lift 100. The patient lift 200 substantially corresponds to the patient lift 100, except the patient lift 200 is configured to emit UV radiation from a UV radiation configuration 240 for exposing a carry bar 210 connected to the strap 124 to the UV radiation. In this respect, the patient lift 200 further includes the UV radiation configuration 240 and the carry bar 210. In some embodiments, for example, the controllable components 106 of the patient lift 200 include the UV radiation configuration 240

As depicted, in some embodiments, for example, the UV radiation configuration 240 is mounted to the housing 120 of the patient lift 200, and configured to emit UV radiation into a radiation zone 242. In some embodiments, for example, the radiation zone 242 is configured such that the carry bar 210 is disposable in the radiation zone 242. In some embodiments, for example, the radiation zone 242 is configured such that the entirety of the carry bar 210 is disposable in the radiation zone 242. In some embodiments, for example, the mounting of the UV radiation configuration 240 to the housing 120 is such that the UV radiation configuration 240 is disposed in the housing 120. In some embodiments, for example, the mounting of the UV radiation configuration 240 to the housing 120 is such that the UV radiation configuration 240 is disposed outside of the housing 120. In some embodiments, for example, the carry bar 210 is disposed outside of the housing 120. In some embodiments, for example, at least a portion of the radiation zone 242 is defined outside of the housing 120. In some embodiments, for example, the entirety of the radiation zone 242 is defined outside of the housing 120. In some embodiments, for example, the radiation zone 242 is defined by the UV radiation configuration 240. In some embodiments, for example, the UV radiation that is emitted by the UV radiation configuration 240 is emitted into the radiation zone 242. In some embodiments, for example, the radiation zone 242 is a zone in which UV radiation that is emitted by the UV radiation configuration 240 is received.

In some embodiments, for example, the strap 124 is connected to the spool 122 and further connected to the carry bar 210. In some embodiments, for example, the carry bar 210 is configured to connect to a patient supporter, such as a sling or a seat.

In some embodiments, for example, the drivability of the spool 122 by the drive unit 126 is such that the carry bar 210 is displaceable, relative to the radiation zone 242. In some embodiments, for example, the displaceability of the carry bar 210, relative to the radiation zone 242, is for effectuating disposition of the carry bar 210 in the radiation zone 242.

In some embodiments, for example, the controller 102 is operably coupled to the UV radiation configuration 240 to adjust a characteristic of the UV radiation (e.g. wavelength and intensity) emitted by the UV radiation configuration 240 into the radiation zone 242, and further operably coupled to the drive unit 126 to adjust the displacement of the carry bar 210, relative to the radiation zone 242.

In some embodiments, for example, similar to the UV radiation configuration 140, a user can input a control command to the controller 102 via the user interface 116 to adjust the characteristics of the UV radiation emitted from the UV radiation configuration 240. In some embodiments, for example, similar to the strap 124, a user can input a control command to the controller 102 via the user interface 116 to adjust the displacement of the carry bar 210, relative to the radiation zone 242.

In some embodiments, for example, while the UV radiation configuration 240 is emitting UV radiation into the radiation zone 242 and the carry bar 210 is disposed in the radiation zone 242, the carry bar 210 is exposed to the UV radiation.

In some embodiments, for example, by exposing the carry bar 210 that is disposed in the radiation zone 242 to the UV radiation emitted by the UV radiation configuration 240, the DNA and RNA of the pathogens and microorganisms disposed on the carry bar 210 are disrupted, with effect that the pathogens and microorganisms become inactive and are unable to reproduce, which, in some embodiments, for example, effectuates disinfecting and sanitizing of the carry bar 210 that is exposed to the UV radiation. The carry bar 210 can be displaced, relative to the radiation zone 242, to move the carry bar 210 in and out of the radiation zone 242, to adjust the duration of exposure of UV radiation to the carry bar 210, and to adjust the minimum spacing distance between the carry bar 210 and the UV radiation configuration 240.

By adjusting the UV radiation wavelength and intensity of the UV radiation emitted by the UV radiation configuration 240 to the carry bar 210, the duration of exposure of UV radiation to the carry bar 210, and the minimum spacing distance between the carry bar 210 and the UV radiation configuration 240, the dosage of UV radiation emitted to the carry bar 210 can be controlled, and therefore, the disinfection and sanitation of the carry bar 210 can be controlled. In some embodiments, for example, the patient lift 200 is configured to deliver the optimal dosage of UV radiation required to achieve effective disinfection of the carry bar 210 without damaging the patient lift 200 itself or posing risks to users.

In some embodiments, for example, by disinfecting and sanitizing the carry bar 210, a safer and more hygienic environment is provided to users, such as patients and healthcare providers.

In some embodiments, for example, the displaceability of the carry bar 210, relative to the radiation zone 242, is for effectuating disposition of the carry bar 210 outside of the radiation zone 242.

In some embodiments, for example, similar to the patient lift 100, the controller 102 is configured to determine a usage frequency of the patient lift 200 based at least in part on the energy provided by the energy source 112 to the drive unit 126. In some embodiments, for example, similar to the patient lift 100, the adjusting of the characteristic of the UV radiation emitted by the UV radiation configuration 240 is based at least in part on the usage frequency of the patient lift 200. In some embodiments, for example, similar to the patient lift 100, the adjusting of the displacement of the carry bar 210, relative to the radiation zone 242, is based at least in part on the usage frequency of the patient lift 200.

In some embodiments, for example, similar to the patient lift 100, the controller 102 is configured to determine that different patients are using the patient lift 200 based at least in part on the energy provided by the energy source 112 to the drive unit 126. In some embodiments, for example, similar to the patient lift 100, the adjusting of the characteristic of the UV radiation emitted by the UV radiation configuration 240 is based at least in part on the determination that different patients are using the patient lift 200. In some embodiments, for example, similar to the patient lift 100, the adjusting of the displacement of the carry bar 210, relative to the radiation zone 242, is based at least in part on the determination that different patients are using the patient lift 200.

In some embodiments, for example, similar to the patient lift 100, the user interface 116 is configured to receive an input representative of an environment of the patient lift 200, for example, from a user via a hand control unit, and communicate the input to the controller 102. In some embodiments, for example, similar to the patient lift 100, the adjusting of the characteristic of the UV radiation emitted by the UV radiation configuration 240 is based at least in part on the environment of the patient lift 200. In some embodiments, for example, similar to the patient lift 100, the adjusting of the displacement of the carry bar 210, relative the radiation zone 242, is based at least in part on the environment of the patient lift 200.

In some embodiments, for example, the user interface 116 is configured to receive an input representative of an operating mode of the patient lift 200, for example, via a hand control unit, and communicate the input to the controller 102. In some embodiments, for example, similar to the patient lift 100, the adjusting of the characteristic of the UV radiation emitted by the UV radiation configuration 240 is based at least in part on the operating mode of the patient lift 200. In some embodiments, for example, the adjusting of the displacement of the carry bar 210, relative to the radiation zone 242, is based at least in part on the operating mode of the patient lift 200.

In some embodiments, for example, the adjustable characteristic of the UV radiation emitted from the UV radiation configuration 240 includes a wavelength of the UV radiation. In some embodiments, for example, the adjusting of the wavelength of the UV radiation includes increasing the wavelength of the UV radiation. In some embodiments, for example, the adjusting of the wavelength of the UV radiation includes decreasing the wavelength of the UV radiation.

In some embodiments, for example, the UV radiation configuration 240 emits broad spectrum UV radiation. In some embodiments, for example, the UV radiation configuration 240 emits UV radiation of various wavelengths to have disinfection efficacy across a wide range of pathogens and microorganisms. In some embodiments, for example, the UV radiation configuration 240 emits Ultraviolet-C (UVC) radiation.

In some embodiments, for example, the adjustable characteristic of the UV radiation emitted from the UV radiation configuration 240 includes an intensity of the UV radiation. In some embodiments, for example, the adjusting of the intensity of the UV radiation includes increasing the intensity of the UV radiation. In some embodiments, for example, the adjusting of the intensity of the UV radiation includes decreasing the intensity of the UV radiation.

In some embodiments, for example, the adjusting of the displacement of the carry bar 210, relative to the radiation zone 242, is to effectuate the disposition of the carry bar 210 in the radiation zone 242. In some embodiments, for example, the adjusting of the displacement of the carry bar 210, relative to the radiation zone 242, is to adjust an amount of time that the carry bar 210 is disposed in the radiation zone 242. In some embodiments, for example, the adjusting of the displacement of the carry bar 210, relative to the radiation zone 242, is such that the displacement of the carry bar 210, relative to the radiation zone 242, is increased. In some embodiments, for example, the adjusting of the displacement of the carry bar 210, relative to the radiation zone 242, is such that the displacement of the carry bar 210, relative to the radiation zone 242, is decreased. In some embodiments, for example, the adjusting of the displacement of the carry bar 210, relative to the radiation zone 242, is such that there is an absence of displacement of the carry bar 210, relative to the radiation zone 242. In some embodiments, for example, the adjusting of the displacement of the carry bar 210, relative to the radiation zone 242, is to adjust a minimum spacing distance between the carry bar 210 and the UV radiation configuration 240. In some embodiments, for example, by reducing the minimum spacing distance between the carry bar 210 and the UV radiation configuration 240, the dosage of the UV radiation emitted by the UV radiation configuration 240 to the carry bar 210 is increased. In some embodiments, for example, by increasing the minimum spacing distance between the carry bar 210 and the UV radiation configuration 240, the dosage of the UV radiation emitted by the UV radiation configuration 240 to the carry bar 210 is decreased. In some embodiments, for example, the adjusting of the displacement of the carry bar 210, relative to the radiation zone 242, is to adjust the dosage of the UV radiation emitted by the UV radiation configuration 240 to the carry bar 210.

In some embodiments, for example, similar to the UV radiation configuration 140, the UV radiation configuration 240 comprises a UV radiation source 244 for emitting UV radiation.

In some embodiments, for example, adjusting the wavelength of the UV radiation emitted from the UV radiation configuration 240 includes adjusting the wavelength emitted from the UV radiation source 244. In some embodiments, for example, adjusting the intensity of the UV radiation emitted from the UV radiation configuration 240 includes adjusting the intensity emitted from the UV radiation source 244.

In some embodiments, for example, the UV radiation source 244 is a first UV radiation source 244, and the UV radiation configuration 240 further comprises a second UV radiation source 244 for emitting UV radiation.

In some embodiments, for example, adjusting the wavelength of the UV radiation emitted from the UV radiation configuration 240 includes adjusting the wavelength of the UV radiation emitted from one or more UV radiation sources 244. In some embodiments, for example, the wavelength of UV radiation emitted from a first UV radiation source 244 is the same as the wavelength of UV radiation emitted from a second UV radiation source 244. In some embodiments, for example, the wavelength of UV radiation emitted from the first UV radiation source 244 and wavelength of UV radiation emitted from the second UV radiation source 244 are different. In some embodiments, for example, adjusting the wavelength of the UV radiation emitted from the UV radiation configuration 240 includes adjusting the wavelength by turning on or off one or more UV radiation sources 244.

In some embodiments, for example, adjusting the intensity of the UV radiation emitted from the UV radiation configuration 240 includes adjusting the intensity of the UV radiation emitted from one or more UV radiation sources 244. In some embodiments, for example, the intensity of UV radiation emitted from the first UV radiation source 244 is the same as the intensity of UV radiation emitted from the second UV radiation source 244. In some embodiments, for example, the intensity of UV radiation emitted from the first UV radiation source 244 and intensity of UV radiation emitted from the second UV radiation source 244 are different. In some embodiments, for example, adjusting the intensity of the UV radiation emitted from the UV radiation configuration 240 includes adjusting the intensity by turning on or off one or more UV radiation sources 244.

In some embodiments, for example, the first UV radiation source 244 and the second UV radiation source 244 are disposed in a side-by-side relationship. In some embodiments, for example, the first UV radiation source 244 and the second UV radiation source 244 are disposed in opposing relationship.

In some embodiments, for example, the adjusting of the displacement of the carry bar 210, relative to the radiation zone 242, is to effectuate the disposition of the carry bar 210, relative to the UV radiation configuration 240, such that the minimum spacing distance between the carry bar 210 and the UV radiation configuration 240 is at its minimum value.

In some embodiments, for example, the patient lift 200 includes a plurality of UV radiation sources 244. In some embodiments, for example, the wavelength and intensity of the UV radiation emitted from the UV radiation configuration 240 are adjusted by turning on the desired UV radiation sources 244, such that the UV radiation emitted from the UV radiation sources 244 combine to generate the UV radiation of desired wavelength and intensity.

In some embodiments, for example, for each one of the UV radiation sources 244, independently, the UV radiation source 244 is a UV light source. In some embodiments, for example, for each one of the UV radiation sources 244, independently, the UV radiation source 244 is a light emitting diode (LED). In some embodiments, for example, the LED is a visible LED. In some embodiments, for example, the LED is a non-visible LED. In some embodiments, for example, for each one of the UV radiation sources 244, independently, the UV radiation source 244 is a UV bulb. In some embodiments, for example, for each one of the UV radiation sources 244, independently, the UV radiation source 244 is a UV radiation emitter.

In some embodiments, for example, the UV radiation configuration 240 is defined by a strip of UV radiation emitters (e.g. UV radiation sources 244).

In some embodiments, for example, similar to the patient lift 100, the patient lift 200 is configured to be connected, for example, mounted, to a ceiling, for example, of a room. In some embodiments, for example, the patient lift 200 is configured to be connected, for example, mounted, to a patient lift supporter, for example, a frame, that is configured to be supported by a reaction surface, such as a floor, for example, of a room. In some embodiments, for example, as depicted in Figure 9, the patient lift 200 is configured to be operably coupled to a track configuration for displacing the patient lift along a first displacement axis. In some embodiments, for example, the track configuration is configured to be connected, for example, mounted, to a ceiling. As depicted, in some embodiments, for example, the track configuration includes a first track 110. In some embodiments, the patient lift 200 and the first track 110 are co-operatively configured such that the patient lift 200 is displaceable, relative to the first track 110, along an axis that is parallel to the longitudinal axis of the first track 110, for displacing the patient lift along the first displacement axis. In some embodiments, for example, the track configuration further includes a second track 170. In some embodiments, the first track 110 and the second track 170 are co-operatively configured such that the first track 110 is displaceable, relative to the second track 170, along an axis that is parallel to the longitudinal axis of the second track 170, for displacing the patient lift 200 along a second displacement axis, wherein the first displacement axis and the second displacement axis are disposed in a non-parallel relationship, for example, a perpendicular relationship. In some embodiments, for example, the track configuration is mountable to a room, for example, the ceiling, via mechanical fasteners (e.g. nuts and bolts, screws, and the like). In some embodiments, for example, the displaceability of the patient lift 200, relative to the track configuration, is effectuatable manually, for example, by applying a displacement force to the patient lift 200.

In some embodiments, for example, the patient lift 200 includes the UV radiation configuration 140 and the UV radiation configuration 240. In some embodiments, for example, the patient lift 200 includes the UV radiation configuration 240 and does not include the UV radiation configuration 140.

In some embodiments, for example, the patient lift 200 includes safety features to oppose, for example, prevent, emission of the UV radiation outside of the radiation zone 142, such that, in some embodiments, for example, the UV radiation is limited to being emitted in the radiation zone 242, for reducing the risk of accidental exposure of the UV radiation to users. In some embodiments, for example, the patient lift 200 includes an emergency pull cord 162, as described herein.

In some embodiments, for example, a user can control the patient lift 200 via a hand control unit of the user interface 116, which is disposed in operable configuration with the controller 102. The user can select the mode that the patient lift 200 is to operate in, and initiate the operation of the patient lift 200. In response to the selection of the operating mode, the wavelength and intensity of the UV radiation emitted by the UV radiation configuration 240 is adjusted, and the displacement of the carry bar 210, relative to the radiation zone 242, is adjusted, for effectuating disposition of the carry bar 210 in the radiation zone 242, establishing the desired minimum spacing distance between the carry bar 210 and the UV radiation configuration 240, and exposing the carry bar 210 with UV radiation having the desired characteristics for the desired duration. The user can further pause or stop the operation of the patient lift 200 via the hand control unit. In some embodiments, for example, after the operation of the patient lift 200 is initiated, a pre-determined amount of time is allowed to pass, before the UV radiation configuration 240 begins to emit UV radiation, which allows time for an operator to leave the room in which the patient lift 200 is disposed. In some embodiments, for example, the status of the patient lift 200 is displayed on the user interface 116, for example, on the hand control unit. In some embodiments, for example, the status of the operation of the patient lift 200 is displayed on the user interface 116, for example, on the hand control unit. In some embodiments, for example, an alert, for example, an audible or visible alert, is provided by the user interface 116, to indicate to a user that the UV radiation configuration 240 is, or will be, emitting UV radiation. In some embodiments, for example, while the UV radiation configuration 240 is emitting UV radiation, the controller 102 will send a control command to the UV radiation configuration 240 to stop emission of the UV radiation after a pre-determined amount of time has passed. In such embodiments, for example, the UV radiation is emitted for the predetermined amount of time to achieve the desired exposure of the carry bar 210 to the UV radiation, and the UV radiation is not emitted more than desired or necessary.

In some embodiments, for example, the patient lift 200 includes visible warnings, such as labels and signs, to indicate to a user or bystander that the patient lift 200 is able to emit UV radiation, and to identify potential hazards of UV radiation and appropriate precautions that can be taken. In some embodiments, for example, the visible warnings are disposed on the housing 120 of the patient lift 200.

In some embodiments, for example, the UV radiation configuration 240 is mountable on other structural components of the patient lift 200 or on an accessory that is connectible to a structural component of the patient lift 200, for providing flexibility in positioning of the UV radiation configuration 240 and radiation zone 242, relative to the carry bar 210, to achieve desirable exposure of the carry bar 210 to the emitted UV radiation, which, in some embodiments, for example, achieves desirable disinfection and sanitization of the carry bar 210.

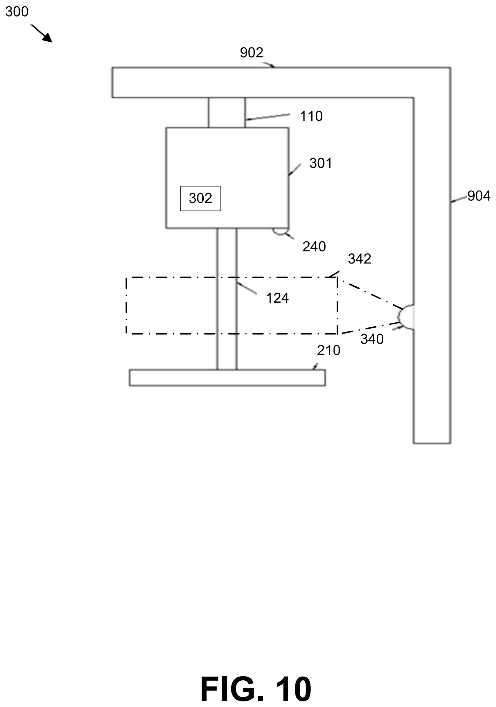

Figure 10 depicts a patient lift configuration 300. The patient lift configuration 300 includes an ultraviolet (UV) radiation configuration 340, mounted to a building structure 900, and configured to emit UV radiation into a radiation zone 342, in a manner substantially similar to the manner by which UV radiation is emitted into the radiation zone 142 or 242 by UV radiation configuration 140 and 240, respectively. In some embodiments, for example, the UV radiation configuration 340 substantially corresponds to the UV radiation configuration 140 and the UV radiation configuration 240 as described herein. In some embodiments, for example, the patient lift configuration 300 includes a patient lift 301. In some embodiments, for example, the patient lift 301 includes a housing 120, a spool 122, a strap 124, a drive unit 126, as described herein. In some embodiments, for example, the patient lift 301 includes a carry bar 210, as described herein. In some embodiments, for example, the patient lift 301 substantially corresponds to the patient lift 100 or to the patient lift 200. In some embodiments, for example, the patient lift 301 substantially corresponds to the patient lift 100 or to the patient lift 200, except the patient lift 301 does not include a UV radiation configuration 140 or the UV radiation configuration 240. In some embodiments, for example, the patient lift 301 includes the UV radiation configuration 140 or the UV radiation configuration 240, or both the UV radiation configuration 140 and the UV radiation configuration 240. In some embodiments, for example, at least a portion of the radiation zone 342 is defined outside of the housing 120 of the patient lift 301. In some embodiments, for example, the entirety of the radiation zone 342 is defined outside of the housing 120 of the patient lift 301. In some embodiments, for example, the patient lift configuration 300 includes a controller 302, substantially similar to the controller 102 described herein. In some embodiments, for example, the controller 302 of the patient lift configuration 300 is the controller of the patient lift 301. The controller 302 is operably coupled to the UV radiation configuration 340 to adjust a characteristic of the UV radiation emitted by the UV radiation configuration 340 into the radiation zone 342 (e.g. wavelength and intensity), and further operably coupled to the drive unit of the patient lift 301 to adjust the displacement of the strap 124 through the radiation zone 342 and to adjust the displacement of the carry bar 210, relative to the radiation zone 342, in a manner substantially to the manner as described with respect to the patient lift 100 and the patient lift 200. Similar to the patient lift 100, while the UV radiation configuration 340 is emitting UV radiation into the radiation zone 342, the portion of the strap 124 that is disposed in the radiation zone 342 is exposed to the UV radiation. Similar to the patient lift 200, while the UV radiation configuration 340 is emitting UV radiation into the radiation zone 342 and the carry bar 210 is disposed in the radiation zone 342, the carry bar 210 is exposed to the UV radiation.

In some embodiments, for example, the patient lift 301 is mounted to a ceiling 902 of the building structure 900, for example, by a track 110. In some embodiments, for example, the UV radiation configuration 340 is mounted to a wall 904 of the building structure 900.

In some embodiments, for example, the user interface of the patient lift configuration 300 is the user interface of the patient lift 302 (e.g. a hand control unit). In some embodiments, for example, the user interface of the patient lift configuration 300 is mounted to the building structure 900, for example, to the wall 904 (e.g. a control panel).

In some embodiments, for example, similar to the patient lift 100 and the patient lift 200, the controller 302 is configured to determine a usage frequency of the patient lift 301 based at least in part on the energy provided by an energy source to the drive unit 126 of the patient lift 301. In some embodiments, for example, similar to the patient lift 100 and the patient lift 200, the adjusting of the characteristic of the UV radiation emitted by the UV radiation configuration 340 is based at least in part on the usage frequency of the patient lift 301. In some embodiments, for example, similar to the patient lift 100 and the patient lift 200, the adjusting of the displacement of the strap 124 through the radiation zone 342 and the adjusting of the displacement of the carry bar 210, relative to the radiation zone 342, are based at least in part on the usage frequency of the patient lift 301.

In some embodiments, for example, similar to the patient lift 100 and the patient lift 200, the controller 102 is configured to determine that different patients are using the patient lift 301 based at least in part on the energy provided by an energy source to the drive unit 126 of the patient lift 301. In some embodiments, for example, similar to the patient lift 100 and the patient lift 200, the adjusting of the characteristic of the UV radiation emitted by the UV radiation configuration 340 is based at least in part on the determination that different patients are using the patient lift 301. In some embodiments, for example, similar to the patient lift 100 and the patient lift 200, the adjusting of the displacement of the strap 124 through the radiation zone 342 and the adjusting of the displacement of the carry bar 210, relative to the radiation zone 342, are based at least in part on the determination that different patients are using the patient lift 301.

In some embodiments, for example, similar to the patient lift 100 and the patient lift 200, the user interface of the patient lift configuration 300 is configured to receive an input representative of an environment of the patient lift configuration 300, for example, from a user via a hand control unit, and communicate the input to the controller 302. In some embodiments, for example, similar to the patient lift 100 and the patient lift 200, the adjusting of the characteristic of the UV radiation emitted by the UV radiation configuration 340 is based at least in part on the environment of the patient lift configuration 300. In some embodiments, for example, similar to the patient lift 100 and the patient lift 200, the adjusting of the displacement of the strap 124 through the radiation zone 342 and the adjusting of the displacement of the carry bar 210, relative the radiation zone 342, are based at least in part on the environment of the patient lift configuration 300.

In some embodiments, for example, similar to the patient lift 100 and the patient lift 200, the user interface of the patient lift configuration 300 is configured to receive an input representative of an operating mode of the patient lift configuration 300, for example, via a hand control unit, and communicate the input to the controller 302. In some embodiments, for example, similar to the patient lift 100 and the patient lift 200, the adjusting of the characteristic of the UV radiation emitted by the UV radiation configuration 340 is based at least in part on the operating mode of the patient lift configuration 300. In some embodiments, for example, similar to the patient lift 100 and the patient lift 200, the adjusting of the displacement of the strap 124 through the radiation zone 342 and the adjusting of the displacement of the carry bar 210, relative to the radiation zone 342, are based at least in part on the operating mode of the patient lift configuration 300.

In some embodiments, for example, the patient lift configuration 300 includes an emergency pull cord 162, as described herein.

In the preceding description, for purposes of explanation, numerous details are set forth in order to provide a thorough understanding of the embodiments; however the specific details are not necessarily required. In other instances, well-known electrical structures and circuits are shown in block diagram form in order not to obscure the understanding. For example, specific details are not provided as to whether the embodiments described herein are implemented as a software routine, hardware circuit, firmware, or a combination thereof.

The steps and/or operations in the flowcharts and drawings described herein are for purposes of example only. There may be many variations to these steps and/or operations without departing from the teachings of the present disclosure. For instance, the steps may be performed in a differing order, or steps may be added, deleted, or modified.

The coding of software for carrying out the above-described methods described for execution by a controller (or processor) or other apparatus is within the scope of a person of ordinary skill in the art having regard to the present disclosure. Machine readable code executable by one or more processors of one or more respective devices to perform the above-described method may be stored in a machine readable medium such as the memory of the data manager. The terms "software" and "firmware" are interchangeable within the present disclosure and comprise any computer program stored in memory for execution by a processor, comprising RAM memory, ROM memory, erasable programmable ROM (EPROM) memory, electrically EPROM (EEPROM) memory, and non-volatile RAM (NVRAM) memory. The above memory types are example only, and are thus not limiting as to the types of memory usable for storage of a computer program.

All values and sub-ranges within disclosed ranges are also disclosed. In addition, although the systems, devices and processes disclosed and shown herein may comprise a specific plurality of elements/components, the systems, devices, configurations, and assemblies may be modified to comprise additional or fewer of such elements/components. For example, although any of the elements/components disclosed may be referenced as being singular, the embodiments disclosed herein may be modified to comprise a plurality of such elements/components. The subject matter described herein intends to cover and embrace all suitable changes in technology.

Although the present disclosure is described, at least in part, in terms of methods, a person of ordinary skill in the art will understand that the present disclosure is also directed to the various components for performing at least some of the aspects and features of the described methods, be it by way of hardware (DSPs, ASIC, or FPGAs), software or a combination thereof. Accordingly, the technical solution of the present disclosure may be embodied in a non-volatile or non-transitory machine readable medium (e.g., optical disk, flash memory, etc.) having stored thereon executable instructions tangibly stored thereon that enable a processing device (e.g., a data manager) to execute examples of the methods disclosed herein.

The term "processor" may comprise any programmable system comprising systems using micro- or nano-processors/controllers, reduced instruction set circuits (RISC), application specific integrated circuits (ASICs), logic circuits, and any other circuit or processor capable of executing the functions described herein. The term "database" may refer to either a body of data, a relational database management system (RDBMS), or to both. As used herein, a database may comprise any collection of data comprising hierarchical databases, relational databases, flat file databases, object-relational databases, object oriented databases, and any other structured collection of records or data that is stored in a computer system. The above examples are example only, and thus are not intended to limit in any way the definition and/or meaning of the terms "processor" or "database".

The present disclosure may be embodied in other specific forms without departing from the subject matter of the claims. The described example embodiments are to be considered in all respects as being only illustrative and not restrictive. The present disclosure intends to cover and embrace all suitable changes in technology. The scope of the present disclosure is, therefore, described by the appended claims rather than by the foregoing description. The scope of the claims should not be limited by the embodiments set forth in the examples, but should be given the broadest interpretation consistent with the description as a whole.

## Claims

1. A patient lift, comprising:
a housing;
an ultraviolet (UV) radiation configuration, mounted to the housing, and configured to emit UV radiation into a radiation zone;
a spool, disposed in the housing;
a strap, connected to the spool, and extending through the radiation zone such that a portion of the strap is disposed in the radiation zone;
a drive unit, operably coupled to the spool such that the spool is drivable by the drive unit for winding the strap onto the spool, and further drivable by the drive unit for unwinding the strap from the spool, such that the strap is displaceable through the radiation zone;
an energy source operably coupled to the drive unit to provide energy to the drive unit;
a controller operably coupled to the UV radiation configuration to adjust a characteristic of the UV radiation emitted by the UV radiation configuration into the radiation zone, and further operably coupled to the drive unit to adjust the displacement of the strap through the radiation zone;
wherein:
while the UV radiation configuration is emitting UV radiation into the radiation zone, the portion of the strap that is disposed in the radiation zone is exposed to the UV radiation.

2. The patient lift of claim 1, wherein:
the controller is configured to determine a usage frequency of the patient lift based at least in part on the energy provided by the energy source to the drive unit; and
the adjusting of the characteristic of the UV radiation is based at least in part on the usage frequency of the patient lift, and the adjusting of the displacement of the strap through the radiation zone is based at least in part on the usage frequency of the patient lift.

3. The patient lift of claim 1 or claim 2, wherein:
the controller is configured to determine that different patients are using the patient lift based at least in part on the energy provided by the energy source to the drive unit; and
the adjusting of the characteristic of the UV radiation is based at least in part on the determination that different patients are using the patient lift, and the adjusting of the displacement of the strap through the radiation zone is based at least in part on the determination that different patients are using the patient lift.

4. The patient lift of any one of claims 1 to 3, further comprising:
a user interface, operably coupled to the controller, and configured to receive an input representative of an environment of the patient lift and to communicate the input to the controller, wherein the adjusting of the characteristic of the UV radiation is based at least in part on the environment of the patient lift, and the adjusting of the displacement of the strap through the radiation zone is based at least in part on the environment of the patient lift.

5. The patient lift of any one of claims 1 to 3, further comprising:
a user interface, operably coupled to the controller, and configured to receive an input representative of an operating mode of the patient lift and to communicate the input to the controller, wherein the adjusting of the characteristic of the UV radiation is based at least in part on the operating mode of the patient lift, and the adjusting of the displacement of the strap through the radiation zone is based at least in part on the operating mode of the patient lift.

6. The patient lift of any one of claims 1 to 5, wherein the adjustable characteristic of the UV radiation includes a wavelength of the UV radiation.

7. The patient lift of any one of claims 1 to 6, wherein the adjustable characteristic of the UV radiation includes an intensity of the UV radiation.

8. The patient lift of any one of claims 1 to 7, wherein the adjusting of the displacement of the strap through the radiation zone is such that another portion of the strap is disposed in the radiation zone.

9. The patient lift of any one of claims 1 to 8, wherein the UV radiation configuration comprises a UV radiation source for emitting UV radiation.

10. The patient lift of any one of claims 1 to 9, wherein:
the strap extends through an opening defined by the housing; and
the opening and the radiation zone are disposed in alignment.

11. The patient lift of any one of claims 1 to 10, wherein:
the strap extends through an opening defined by an opening-defining surface of the UV radiation configuration; and
at least a portion of the opening-defining surface of the UV radiation configuration is disposed in the radiation zone, such that, while the UV radiation configuration is emitting the UV radiation into the radiation zone, the at least a portion of the opening-defining surface of the UV radiation configuration is exposed to the UV radiation.

12. The patient lift of any one of claims 1 to 11, wherein the patient lift is configured to be connected to a ceiling.

13. The patient lift of any one of claims 1 to 11, wherein the patient lift is configured to be connected to a patient lift supporter that is configured to be supported by a reaction surface.

14. The patient lift of any one of claims 1 to 11, wherein the patient lift is configured to be operably coupled to a track configuration for displacing the patient lift along a displacement axis.

15. The patient lift of any one of claims 1 to 14, wherein the mounting of the UV radiation to the housing is such that the UV radiation configuration is disposed in the housing, such that the UV radiation that is emitted by the UV radiation configuration is opposed by the housing from being emitted outside of the housing.
